# EUROPÄISCHE PATENTANMELDUNG

(11) **EP 4 809 965 A2**
(43) Veröffentlichungstag der Anmeldung: **23.09.2026**
(21) Anmeldenummer: 26185035.8
(22) Anmeldetag: 26.06.2025
(51) Int. Cl.: A61B 90/98

(54) **VERFAHREN ZUM MARKIEREN EINES OBJEKTES MIT EINEM FUNKETIKETT UND MIT EINEM FUNKETIKETT MARKIERTES OBJEKT**

(30) Priorität: 26.06.2024 DE 102024118144
(62) Dokumentnummer(n) der früheren Anmeldung(en) nach Art. 76 EPÜ: 25739715.8 / 4 805 853
(71) Anmelder: Asanus Medizintechnik GmbH, 78579 Neuhausen ob Eck (DE)
(72) Erfinder: SCHORER, Armin, 78597 Irndorf (DE)
(74) Vertreter: Kreutzer, Ulrich

(57) **Zusammenfassung**

Die Erfindung betrifft ein Verfahren zum Markieren eines Objektes mit einem Funketikett und ein mit einem Funketikett (60) markiertes Objekt, das einen Abschnitt (50) aufweist, in dem eine Durchgangsbohrung (52) mit einem Durchgangsdurchmesser und zwei einander gegenüberliegenden Öffnungen (56, 58) vorgesehen ist. Bei dem Objekt ist wenigstens eine erste (56) der beiden Öffnungen (56, 58) kegelförmig gegenüber dem Durchgangsdurchmesser verbreitert. Ein Funketikett (60) ist zumindest partiell in der Durchgangsbohrung (52) angeordnet, wobei das Funketikett (60) und die Durchgangsbohrung (52) einschließlich ihrer Öffnungen (56, 58) und jeder kegelförmigen Verbreiterung (62) mit einer ausgehärteten Vergussmasse vergossen ist, die eine Schutzschicht (64) über dem Funketikett (60) bildet.

## Beschreibung

### TECHNISCHES GEBIET DER ERFINDUNG

Die Erfindung betrifft ein Verfahren zum Markieren eines Objektes, insbesondere eines metallischen Objektes wie eines Werkzeugs, Implantats, Bauteils, Schmuckstücks, medizinischen Instruments etc. mit einem Funketikett. Die Erfindung betrifft auch ein mit einem Funketikett markiertes Objekt, insbesondere ein metallisches Objekt wie ein Werkzeug, Implantat, Bauteil, Schmuckstück, medizinisches Instrument etc.

### HINTERGRUND DER ERFINDUNG

Aus unterschiedlichsten Gründen kann es wünschenswert sein, Objekte der genannten Art und vieles mehr mit einer Markierung zu versehen. So ist es z.B. im medizinischen Bereich aufgrund der stark gestiegenen Anforderungen an die Dokumentationspflicht bei Operationen extrem wichtig, genau dokumentieren zu können, welche medizinischen Instrumente bei einer bestimmten Operation eingesetzt wurden. Gerade auf diesem Gebiet bietet die RFID-Technologie große Möglichkeiten zur automatischen Erfassung der verwendeten Instrumente, Implantate und sonstigen medizinischen Gerätschaften, wenn diese mit hier allgemein und nicht-beschränkend als Funketiketten bezeichneten RFID-Tags, die in unterschiedlichster Bauart bekannt sind, markiert sind. Insbesondere erlauben es z.B. die von der Anmelderin vorgestellten Systeme und Verfahren, automatisch eine ganze Reihe von Informationen über den Operationsverlauf zu erfassen und insbesondere auch sicherzustellen, dass, was erstaunlich häufig vorkommt, keine Instrumente unbeabsichtigt im Patienten verbleiben.

Bei der Markierung von Objekten mit Funketiketten bestehen zwei große Probleme, nämlich erstens die dauerhafte Anbringung eines Funketiketts an dem zu markierenden Objekt, insbesondere wenn es sich dabei um ein Objekt wie ein medizinisches Instrument handelt, das starken Materialbelastungen z.B. beim Reinigen, Desinfizieren und Sterilisieren ausgesetzt ist., und zweitens die Gewährleistung einer guten Auslesbarkeit des Funketiketts, insbesondere wenn es sich um ein metallisches Objekt handelt, das an sich die zum Auslesen des Funketikettes verwendete elektromagnetischen Wellen abschirmt.

Es wurden schon verschiedene Vorschläge gemacht, ein Funketikett an einem Objekt wie einem medizinischen Instrument anzubringen. So schlagen die WO 2009/063503 A2 und die WO 2010/145651 A2 jeweils vor, das Funketikett in einen sogenannten "Plug" (eine Art Kunststoffstöpsel) einzugießen, der das Funketikett hervorragend vor Umwelteinflüssen schützt, und den Plug dann in eine Aufnahmebohrung einzupressen, wo er im Wesentlichen reibschlüssig gehalten wird. Dabei wird der Plug unter große Spannung gesetzt, was zusammen mit den z.B. beim Autoklavieren auftretenden hohen Temperaturen zu einer besonderen Materialbelastung und erhöhtem Verschleiß führt.

Aus der WO 2011/054355 A2 ist ein Verfahren zum Aufkleben eines Funketiketts auf ein zu markierendes Objekt wie insbesondere ein medizinisches Instrument bekannt, mit dem ein Funketikett vorteilhaft mit einer Schutzschicht auf dem zu markierenden Objekt überzogen wird, die eine Doppelfunktion erfüllt: zum einen befestigt sie das Funketikett auf dem Objekt, zum anderen schützt sie das Funketikett vor Umwelteinflüssen und bildet gleichzeitig einen schmutzkantenfreien Übergang zum Objekt. Da das Funketikett aufgeklebt wird, steht es von der Fläche, auf die es aufgeklebt ist, vor, was allerdings in bestimmten Anwendungsfällen unerwünscht sein und zudem die ästhetische Wirkung beeinträchtigen kann. Aus der WO 2015/086775 A1 ist ein ganz ähnliches Verfahren bekannt, bei dem ebenfalls ein Funketikett aufgeklebt wird. Die Funketiketten werden bei beiden Verfahren allein durch die Adhäsionswirkung der Schutzschicht gehalten.

Aus der US 7,837,694 B2 ist es unter anderem bekannt, in eine Kante eines zu markierenden metallischen Objektes eine Ausnehmung einzubringen, so dass sich eine stufenartige, zu zwei zueinander im Wesentlichen orthogonal verlaufenden Seiten hin offene Aussparung ergibt, in die ein Funketikett eingelegt und vergossen werden kann.

Dabei sei bereits an dieser Stelle darauf hingewiesen, dass unter "Vergießen" hier allgemein das Ausfüllen von Freiräumen zwischen dem zu markierenden Objekt und dem Funketikett mit einer aushärtbaren Vergussmasse verstanden wird, wobei die Vergussmasse von selbst aushärten kann oder das Aushärten initiiert sein kann, also z.B. durch Einstrahlen von Licht bestimmter Wellenlängen ausgelöst werden kann.

Weiter sei darauf hingewiesen, dass unter "zu einer, zwei oder mehr Seiten offen" hier verstanden wird, dass die so bezeichnete Ausnehmung, Aussparung, Nut etc. in dem Objekt von einer, zwei bzw. drei Seiten des Objektes aus zugänglich ist, wobei insbesondere bei runden Objekten der Begriff "Seite" im Sinne von Richtungen zu verstehen ist: wenn das Objekt vor einem Betrachter (oder einer Antenne zum Auslesen von Funketiketten) liegt, wäre eine Seite z.B. die dem Betrachter zugewandte Seite, eine zwei Seite die dem Betrachter in Blickrichtung genau abgewandte Seite und eine dritte Seite eine aus einer zur aktuellen Blickrichtung orthogonalen Richtung betrachtete Seite. Handelte es sich bei dem Objekt um einen Standardspielwürfel, bei dem sich die sechs Zahlen auf gegenüberliegenden Seiten jeweils zu sieben ergänzen, wäre eine erste Seite z.B. die Seite mit der Zahl eins, eine zweite Seite die Seite mit der Zahl zwei und eine dritte Seite die Seite mit der Zahl sechs.

Die aus der US 7,837,694 B2 bekannte zu zwei zueinander im Wesentlichen orthogonal verlaufenden Seiten hin offene Aussparung erhöht zwar die Lesbarkeit eines in die Aussparung eingebrachten Funketiketts, problematisch bleibt jedoch dessen dauerhafte Befestigung, insbesondere wenn das zu markierendes Objekt und die Vergussmasse unterschiedliche Wärmeausdehnungskoeffizienten besitzen.

Zur zumindest partiellen Lösung dieses Problems schlägt die US 7,722,531 B1 das Einbringen einer zu einer Seite bzw. zu zwei Seiten offenen Ausnehmung mit einer verhältnismäßig komplizierten Form in das zu markierende Objekt vor, um in dieser Ausnehmung Vergussmasse dauerhaft formschlüssig zu verankern. Allerdings sind die vorgeschlagenen Ausnehmungsformen komplex, so dass sie nicht einfach in ein zu markierendes Objekt wie ein medizinisches Instrument einzubringen sind.

Ein weiterer Ansatz zur Lösung der genannten Probleme wird in der WO 2020/125865 A1 vorgestellt, die das Einbringen verschiedener Vor- und/oder Rücksprünge in die Seitenwände der hinterschnittenen Nut zeigt. Ähnliche Ansätze sind aus der WO 2019/020693 A1 und der JP 2007 208294 A bekannt. Allerdings hat sich gezeigt, dass das bei metallischen Instrumenten in der Regel nach dem Ausbilden der hinterschnittenen Nut erfolgende Einbringen der Vor- und/oder Rücksprünge mechanisch aufwendig und einen gesonderten Arbeitsschritt erfordert. Zudem hat sich gezeigt, dass bei bestimmten Vor- und/oder Rücksprüngen die damit bewirkte formschlüssige Halterung nicht immer ausreicht, den in der Nut durch Vergießen gebildeten Vergusskörper bei Auftreten von Kräften in der Längsrichtung in der Nut zu halten.

Aus der JP 2019-185494 A ist ein Ansatz zur Lösung der genannten Probleme bekannt, bei dem mittels einer aufwendigen Laserbearbeitung der Boden mit einer Vielzahl von zufällig geformten kleinen Öffnungen versehen wird. Es hat sich jedoch gezeigt, dass bestimmte eher zähe Vergussmassen, die besonders zur Markierung medizinischer Instrumente geeignet sind und die den hohen Beanspruchungen, die zum Beispiel beim Autoklarvieren auftreten, dauerhaft standhalten, nicht in die Öffnungen eindringen und der nach Aushärten der Vergussmassen gebildete Vergusskörper nicht ausreichend in dem zu markierenden Objekt verankert wird.

Aus der EP 3 064 293 A1 ist ein Gusskörper mit einem Kennzeichnungselement und einem Haltekörper bekannt, der das Kennzeichnungselement positioniert, der wenigstens zum Teil in das Gussmaterial des Gusskörpers eingegossen ist und einen freiliegenden Kühlbereich aufweist, wobei das Kennzeichnungselement und der Haltekörper bezüglich des Materials verschiedene Beschaffenheit aufweisen und wobei der Haltekörper das Kennzeichnungselement wenigstens teilweise umgibt. Damit wird eine thermisch schonende Behandlung des Kennzeichnungselementes während des Gussprozesses ermöglicht.

Aus der DE 10 2021 106 454 A1 ist ein Schutzgehäuse zum Unterbringen eines darin aufnehmbaren Transponders in einem medizinischen Produkt, insbesondere in einem chirurgischen Instrument und/oder Medizinprodukt in einem Sterilisationskreislauf, bekannt. Dabei weist das Schutzgehäuse an zwei gegenüberliegenden Seiten mindestens zwei integrierte sich diagonal gegenüberliegende Rastnasen auf, die ausgebildet sind, um in Einkerbungen innerhalb des medizinischen Produkts hinterschneidend einzurasten. Das Schutzgehäuse ist länglich ausgebildet und weist entlang der Längsachse eine kreisförmige Durchgangsbohrung zur Aufnahme eines Transponders auf. Allerdings erfordert diese Lösung ein separates Schutzgehäuse mit Rastnasen und entsprechende Einkerbungen in dem medizinischen Produkt, was den Fertigungsaufwand erhöht.

Aus der EP 2 474 941 B1 ist ein RFID-Etikettenhalter bekannt, der verwendet wird, um ein RFID-Etikett in einem Anordnungs-Loch einer Vorrichtung anzuordnen, wobei das Anordnungs-Loch aus einem magnetisch befestigbaren Material gefertigt ist. Der RFID-Etikettenhalter umfasst einen RFID-Etikettenträger, ein Metallsubstrat mit zwei Durchgangslöchern, eine Basis mit einer kreisförmigen Fläche und wenigstens einer magnetischen Komponente, wobei die Form der kreisförmigen Fläche an die Form des Anordnungs-Loches angepasst ist, und die magnetische Komponente verwendet wird, um das Anordnungs-Loch magnetisch befestigbar zu machen.

Aus der EP 1 637 311 A2 ist ein Verfahren zum Aufbringen eines elektronischen Etiketts auf einen durch Tiefziehen hergestellten Gegenstand bekannt, wobei das elektronische Etikett in die Kavität einer Tiefziehform eingesetzt und danach der Gegenstand durch Tiefziehen einer erwärmten Folie hergestellt wird, wobei während des Tiefziehens der Folie das elektronische Etikett mit dem Umfang des tiefgezogenen Gegenstands verbunden wird.

Aus der DE 698 14 890 T2 ist eine Vorrichtung zur Anpassung einer Vorrichtung mit stereotaktischen und endoskopischen Mitteln bekannt. Die Kugel, die ein Kugelgelenk bilden wird, ist mit einem durchlaufenden Kanal zum Einfügen medizinischer Instrumente ausgestattet. In einer bevorzugten Ausführungsform ist die Kugel mit einem sich zentral erstreckenden, eingegossenen Rohr zum Führen des Instruments durch die Kugel ausgestattet. Allerdings betrifft diese Lösung eine stereotaktische Vorrichtung zur Positionierung von Instrumenten während einer Operation und nicht die dauerhafte Markierung von Objekten mit Funketiketten.

### OFFENBARUNG DER ERFINDUNG

Der Erfindung liegt die Aufgabe zugrunde, ein verbessertes Verfahren zum Markieren eines Objektes mit einem Funketikett anzugeben, das es einerseits ermöglicht, das Funketikett möglichst spannungsfrei an dem Objekt zu befestigen, dass das Funketikett sowohl sehr gut auslesbar als auch sicher vor Herausfallen und vor Umwelteinflüssen geschützt ist, das aber andererseits auch effizient mit geringem Fertigungsaufwand anwendbar sein soll. Der Erfindung liegt auch die Aufgabe zugrunde, ein mit einem Funketikett markiertes Objekt anzugeben, bei dem das Funketikett in der besagten Weise an dem Objekt angebracht ist. Unter einem besonderen Aspekt der Erfindung soll ermöglicht werden, das Funketikett bündig an dem Objekt anzubringen, d.h. ohne von der eigentlichen Objektoberfläche vorzuspringen und mithin die ästhetische Wirkung des markierten Objektes nicht zu beeinträchtigen.

Die Aufgabe wird gelöst von einem Verfahren mit den Merkmalen des Anspruchs 1 bzw. einem Objekt mit den Merkmalen des Anspruchs 5. Die jeweiligen Unteransprüche betreffen vorteilhafte Ausgestaltungen und Weiterbildungen.

Bei einem alternativen Verfahren, das nicht Gegenstand dieser Anmeldung ist, wird zunächst eine in einer Längsrichtung durchgehende, zu drei Seiten offene hinterschnittene Nut mit zwei zueinander geneigten Seitenwänden und einem Boden in einem Abschnitt des zu markierenden Objektes ausgebildet, und zwar derart, dass der Boden der Nut wenigstens zwei in der Längsrichtung gegenläufige Gefälle aufweist. Dann wird das Funketikett in die Nut eingebracht, und die Nut wird mit einer aushärtbaren Vergussmasse ausgegossen. Nach Aushärten der Vergussmasse bildet sich dann in der Nut ein dort fest verankerter Vergusskörper mit dem Funketikett, das gut auslesbar ist, wobei der Aufwand zum Ausbilden der Nut gering ist. Mit einem geeigneten Werkzeug lässt sich die entsprechend ausgestaltete Nut in einem Arbeitsgang bilden. Die Längsrichtung ist dabei die Richtung zwei einander gegenüberliegenden Seiten der drei Seiten, zu denen hin die Nut offen ist. Wäre die Nut in der entsprechenden Weise in den oben genannten Standardspielwürfel eingebracht und zu den Seiten mit den Zahlen eins, zwei und sechs hin offen, wäre die Längsrichtung die Richtung zwischen den Seiten mit den Zahlen eins und sechs, wobei es, wie für den Fachmann ersichtlich, unwichtig ist, ob die Richtung als von der Seite mit der Zahl eins zur Seite mit der Zahl sechs hin laufend oder umgekehrt gesehen wird.

Bei einer bevorzugten Ausführungsform des alternativen Verfahrens werden die zwei gegenläufigen Gefälle von wenigstens zwei der Längsrichtung versetzt nebeneinander verlaufenden Wellenbahnen gebildet. Eine solche Ausgestaltung erlaubt es der Vergussmasse in vorteilhafter Weise, entlang der Wellenbahnen zu fließen und die Nut vorteilhaft vollständig auszufüllen.

Bei einer weiteren bevorzugten Ausführungsform des alternativen Verfahrens werden die gegenläufigen Gefälle so ausgebildet, dass eine in der Längsrichtung auf den Vergusskörper aufgebrachte Kraft ein Drücken des Vergusskörpers gegen die zueinander geneigten Seitenwände bewirkt. Es hat sich gezeigt, dass bei bestimmten automatisierten Vorgängen, zum Beispiel der Reinigung medizinischer Instrumente, teilweise sehr hohe Kräfte auf den Vergusskörper in der Längsrichtung der Nut ausgeübt werden. Die Ausgestaltung bewirkt, dass solche Kräfte in Kräften resultieren, die sowohl Anteile in der Längsrichtung der Nut als auch senkrecht dazu besitzen, wobei die senkrechten Anteile der Kräfte dann den Vergusskörper gegen die schräg zueinander geneigten Seitenwände der Nut pressen und entsprechend den Vergusskörper zwischen den Seitenwänden und dem Boden der Nut fester verankern.

Das genannte alternative Verfahren kann vorteilhaft so durchgeführt werden, dass das Funketikett beim Einbringen in die Nut in der Nut angeklebt wird. Dies kann mit einem oder wenigen Tropfen der Vergussmasse oder einem gesonderten Klebstoff erfolgen. Das Ankleben ermöglicht es, bestimmte Verfahrensschritte weiter zu automatisieren, da das Funketikett dann provisorisch in der Nut fixiert ist und nicht sofort vergossen werden muss.

Bei einer weiteren vorteilhaften Ausführungsform des alternativen Verfahrens wird das Funketikett vor dem Einbringen in die Nut in einen Hüllkörper eingebracht und in selbigem dann in die Nut eingebracht. Ein solches Vorgehen hat verschiedene Vorteile. So kann der Hüllkörper die Handhabung der in der Regel sehr kleinen Funketiketten erleichtern, z.B. ein maschinelles Greifen und Einbringen selbiger in die Nut. Zudem bewirkt der Hüllkörper, dass entsprechend weniger Volumen an Vergussmasse in die Nut zum Ausgießen selbiger eingebracht werden muss, was den Vorgang des Ausgießens erleichtert und beschleunigt. Generell gilt zudem, dass je weniger Vergussmasse in eine Öffnung eingebracht wird, um so geringer die Chancen für eine ungewünschte Blasenbildung sind.

Wird ein Hüllkörper verwendet, kann bei dem alternativen Verfahren vorteilhaft so vorgegangen werden, dass der Hüllkörper mit dem Funketikett so in die Nut eingebracht wird, dass sich das Funketikett in einem dem Boden der Nut zugewandten Abschnitt des Hüllkörpers befindet. Es hat sich nämlich gezeigt, dass es das Auslesen des Funketiketts begünstigt, wenn sich dieses nahe dem Boden der Nut befindet.

Bei einem erfindungsgemäßen Verfahren zum Markieren eines Objektes mit einem Funketikett wird eine Durchgangsbohrung mit einem Durchgangsdurchmesser und zwei einander gegenüberliegenden Öffnungen in einem Abschnitt des zu markierenden Objektes ausgebildet, und wenigstens eine erste der beiden Öffnungen wird zur kegelförmigen Verbreiterung der ersten Öffnung gegenüber dem Durchgangsdurchmesser gesenkt. Dabei ist für den Fachmann offensichtlich, dass das sog. Senken, also das kegelartigen Erweitern einer Bohrung auch vor dem Einbringen der eigentlichen Durchgangsbohrung oder in einem Arbeitsgang zusammen mit dem Einbringen selbiger erfolgen kann, wenn das Werkzeug entsprechend ausgebildet ist. Auch ist klar, dass die Durchgangsbohrung nicht im klassischen Sinne gebohrt sein muss, sondern auch in anderer für das jeweilige Objekt geeigneter Weise eingebracht werden kann, z.B. durch Laserschneiden oder Stanzen. Sodann wird ein Funketikett zumindest partiell in der Durchgangsbohrung angeordnet, und das Funketikett, die Durchgangsbohrung einschließlich ihrer Öffnungen und jeder kegelförmigen Verbreiterung wird mit einer aushärtbaren Vergussmasse unter Ausbildung einer Schutzschicht über dem Funketikett ausgegossen. Sodann wird die Vergussmasse zur Bildung eines Vergusskörpers ausgehärtet.

Bei einer Ausführungsform des erfindungsgemäßen Verfahrens werden beide Öffnungen zur kegelförmigen Verbreiterung gesenkt.

Bei einer weiteren Ausführungsform des erfindungsgemäßen Verfahrens wird das Funketiketts in oder über der zweiten der beiden Öffnungen derart angeordnet, dass in Richtung der Durchgangsbohrung gesehen wenigstens ein Abschnitt des Funketiketts über den Durchgangsdurchmesser hinausragt.

Bei einer weiteren Ausführungsform des erfindungsgemäßen Verfahrens wird die Schutzschicht kugelsegmentartig über dem Funketikett ausgebildet.

Das erfindungsgemäße Verfahren löst die genannte Aufgabe und stellt insbesondere dann eine vorteilhafte Lösung dar, wenn das Objekt aufgrund seiner Form und/oder Materialeigenschaften das Einbringen der zu drei Seiten hin offenen Nut nicht gestattet. Das Verfahren kann einen Schritt des Planschleifens etwaig aus der ersten Öffnung vorstehender ausgehärteter Vergussmasse umfassen.

Ein mit einem Funketikett markiertes Objekt, das nicht Gegenstand der vorliegenden Anmeldung ist, weist einen Abschnitt auf, in dem eine in einer Längsrichtung durchgehende, zu drei Seiten offene hinterschnittene Nut mit zwei zueinander geneigten Seitenwänden und einem Boden ausgebildet ist, wobei der Boden der Nut wenigstens zwei in der Längsrichtung gegenläufige Gefälle aufweist und wobei die Nut mit einem Vergusskörper mit dem Funketikett ausgefüllt ist.

Bei einer Ausführungsform des vorgenannten Objekts sind die wenigsten zwei gegenläufigen Gefälle von wenigstens zwei in der Längsrichtung versetzt nebeneinander verlaufenden Wellenbahnen gebildet, was die oben bereits genannten Vorteile besitzt.

Bei einer weiteren Ausführungsform des vorgenannten Objekts ist das Funketikett in einem Hüllkörper in die Nut eingebracht. Bei einem solchen Hüllkörper kann es sich zum Beispiel um ein Spritzgussteil mit einer speziell zur Aufnahme der jeweils verwendeten Funketiketten ausgebildeten Ausnehmung handeln, wobei die Ausnehmung vorteilhaft mit Vorsprüngen versehen sein kann, die ein in die Ausnehmung eingebrachtes Funketikett in der Ausnehmung zurückhalten, so dass es dort nicht weiter verklebt werden muss.

Bei einer weiteren Ausführungsform des vorgenannten Objekts ist der Hüllkörper mit einer Ausrichtmarkierung versehen, die eine Vorzugsrichtung beim Einbringen des Hüllkörpers in die Nut angibt. Eine solche Markierung kann leicht nicht nur von einem Menschen, sondern auch von einer Maschine erkannt werden und nicht nur dazu beitragen, eine gewünschte Ausrichtung des Funketiketts in der Nut zu gewährleisten, sondern den Fertigungsvorgang weiter zu automatisieren

Nach dem Aushärten der Vergussmasse geht diese eine nicht bloß reibschlüssige oder allein auf Adhäsionskräften beruhende, sondern eine formschlüssige Verbindung mit dem Objekt ein. Bei den typischerweise verwendeten Vergussmassen ist die Verbindung bei Zimmertemperatur praktisch spannungsfrei und kann daher Belastungen, wie sie z.B. beim Autoklavieren aufgrund unterschiedlicher Wärmeausdehnungskoeffizienten der Vergussmasse und des Objektes auftreten können, besser kompensieren, als wenn sie ständig unter Spannung stünde. Zudem hat sich gezeigt, dass die vorgestellte Art der Anbringung sehr kosteneffizient vorgenommen werden kann und dass bei bestimmten Ausgestaltungen nur ein einziges Werkzeug zum Ausbilden einer hinterschnittenen Nut und Erzeugen des wenigstens einen Vor- oder Rücksprungs in der Längsrichtung der Nut ausreicht.

Gemäß einem weiteren Aspekt betrifft die Erfindung auch ein mit einem Funketikett markiertes Objekt, das einen Abschnitt aufweist, in dem eine Durchgangsbohrung mit einem Durchgangsdurchmesser und zwei einander gegenüberliegenden Öffnungen vorgesehen ist, wobei wenigstens eine erste der beiden Öffnungen kegelförmig gegenüber dem Durchgangsdurchmesser verbreitert ist, ein Funketikett zumindest partiell in der Durchgangsbohrung angeordnet ist, und das Funketikett und die Durchgangsbohrung einschließlich ihrer Öffnungen und jeder kegelförmigen Verbreiterung mit einer ausgehärteten Vergussmasse vergossen ist, die eine Schutzschicht über dem Funketikett bildet.

Bei einer bevorzugten Ausführungsform des Objektes sind beide Öffnungen gegenüber dem Durchgangsdurchmesser kegelförmig verbreitert.

Bei einer bevorzugten Ausführungsform des Objektes ist das Funketiketts in oder über der zweiten der beiden Öffnungen derart angeordnet, dass in Richtung der Durchgangsbohrung gesehen wenigstens ein Abschnitt des Funketiketts über den Durchgangsdurchmesser hinausragt.

Bei einer bevorzugten Ausführungsform des Objektes ist die Schutzschicht kugelsegmentartig über dem Funketikett ausgebildet.

Weitere Einzelheiten und Vorteile der Erfindung ergeben sich aus der nachfolgenden rein beispielhaften und nicht-beschränkenden Beschreibung von Ausführungsbeispielen in Verbindung mit der 15 Zeichnungsfiguren umfassenden Zeichnung.

### KURZE BESCHREIBUNG DER ZEICHNUNG

- Fig. 1: zeigt eine Seitenansicht eines Abschnitt eines Objektes, hier einer Schere, mit einer nicht-erfindungsgemäß ausgebildeten Nut zur Einbringung eines Funketiketts.
- Fig. 2: zeigt den Abschnitt gemäß Fig. 1, gesehen in Richtung des Pfeiles II in Fig. 1 von unten auf den Abschnitt des Objektes.
- Fig. 3: zeigt einen Schnitt entlang der Linie III-III gemäß Fig. 1.
- Fig. 4: zeigt einen Schnitt entlang der Linie IV-IV gemäß Fig. 1.
- Fig. 5: zeigt den durch den Kreis V in Fig. 2 markierten Abschnitt in vergrößertem Maßstab.
- Fig. 6: zeigt den durch den Kreis VI in Fig. 3 markierten Abschnitt in vergrößertem Maßstab.
- Fig. 7: zeigt den durch den Kreis VII in Fig. 4 markierten Abschnitt in vergrößertem Maßstab.
- Fig. 8: zeigt den durch den Kreis VIII in Fig. 1 markierten Abschnitt in vergrößertem Maßstab.
- Fig. 9: zeigt einen Abschnitt eines zu markierenden Objektes in einer Draufsicht ähnlich Fig. 8 auf eine erste Seite des Abschnittes.
- Fig. 10: zeigt eine Draufsicht auf die der ersten Seite des Abschnitts gemäß Fig. 9 gegenüberliegende Seite.
- Fig. 11: zeigt einen Hüllkörper in schematisierter Darstellung.
- Fig. 12: zeigt ähnlich Fig. 8 eine Seitenansicht eines Abschnitt eines Objektes mit einer Nut gemäß einer weiteren Ausführungsform, die nicht Gegenstand dieser Anmeldung ist.
- Fig. 13: zeigt eine Draufsicht auf einen Abschnitt eines erfindungsgemäß markierten Objektes.
- Fig. 14: zeigt einen Schnitt entlang der Linie XIV-XIV gemäß Fig. 13.
- Fig. 15: zeigt den Abschnitt des Objektes gemäß Fig. 14 ohne Funketikett und ohne Vergussmasse.

### BESCHREIBUNG BEVORZUGTER AUSFÜHRUNGSFORMEN

In den Zeichnungen bezeichnen identische Bezugszeichen gleichwirkende Teile, und es wurde darauf verzichtet, bei jeder Figur alle Bezugszeichen gesondert zu erwähnen. Am Ende dieser Beschreibung ist eine Bezugszeichenliste gegeben, der die Bedeutung der Bezugszeichen entnommen werden kann.

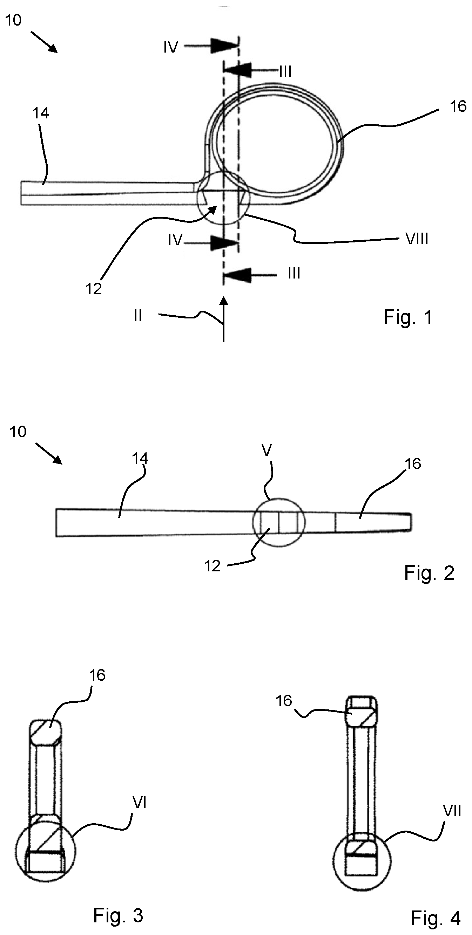

In den Figuren 1 bis 8 sind unterschiedliche Teile eines Abschnitt 10 eines zu markierenden Objektes gezeigt, in dem eine hinterschnittene Nut 12 ausgebildet ist. Bei dem Objekt handelt es sich zum Beispiel um eine chirurgische Schere, bei dem Abschnitt um einen Griffabschnitt der Schere mit Schenkel 14 und Auge 16. Die zu markierenden Objekte sind typischerweise aus Metall, insbesondere Edelstahl, gefertigt.

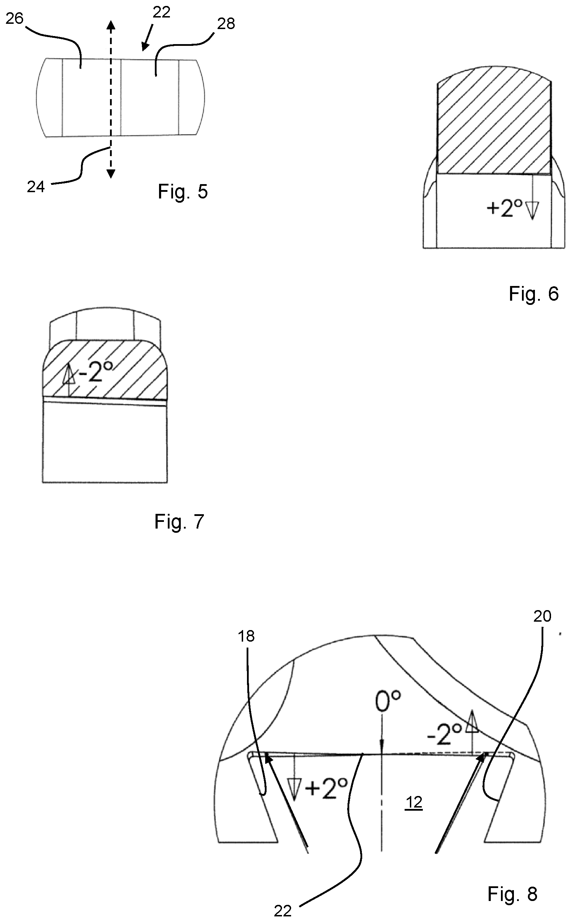

Die Nut 12 ist zu drei Seiten hin offen, und zwar in Fig. 1 zu der dem Betrachter zugewandten Seite des Abschnittes 10, zu der dieser Seite gegenüberliegenden, vom Betrachter abgewandten Seite und nach unten. Wie in Fig. 8 gut zu erkennen, wird die Nut begrenzt von zwei zueinander geneigten Seitenwänden 18 und 20 und einem Boden 22. Anders ausgedrückt ist die in einer Längsrichtung durchgehende, zu drei Seiten offene hinterschnittene Nut 12 zu einer ersten Seite des Abschnittes 10 des Objektes hin offen und in der Draufsicht auf diese Seite hinterschnitten, wobei sich die Nut in dieser Draufsicht in einer zur Blickrichtung orthogonalen Richtung erstreckt und an ihren beiden Enden offen ist.

Zu dem in den Figuren 1 bis 8 dargestellten Zeitpunkt ist noch kein Funketikett in die Nut 12 eingebracht. Dieses wird wie nachfolgend noch erläutert in die Nut eingebracht, und die Nut wird dann mit einer aushärtbaren Vergussmasse vollständig ausgegossen. Bei dieser Vergussmasse kann es sich in an sich bekannter Weise um ein Gemisch aus polymerisierbaren Acrylaten oder Methacrylaten oder eines festen teilpolymerisierten Gemischs aus polymerisierbaren Acrylaten oder Methacrylaten handeln. Die Polymerisierung führt zur Aushärtung des Gemischs. Vorzugsweise handelt es sich um ein Gemisch, dessen Polymerisation durch Bestrahlung mit Licht, z.B. mit einer Aktivierungswellenlänge von 400 - 500 nm, gestartet werden kann. Dazu kann das Gemisch einen Fotoinitiator umfassen, der z.B. aus der aus Benzophenon, Benzoin, einem Alpha-Diketon, Akylphospinoxid, Campferchinon und deren Derivaten sowie Gemischen dieser Fotoinitiatoren bestehenden Gruppe gewählt ist. Alternativ kann das Gemisch auch so ausgebildet sein, dass die Aushärtung durch Anwendung von Druck und/oder Temperatur erfolgt.

Die Monomehreinheiten des Gemisches werden vorzugsweise aus der Gruppe gewählt, die durch Tetraethylenglycoldimethacrylat, Diethylenglycoldimethacrylat, Ethylenglycoldimethacrylat, Polyethylenglycoldimethacrylat bildende Monomere, Butandioldimethacrylat, Hexandioldimethacrylat, Decandioldimethacrylat, Dodecandioldimethacrylat, Bisphenol-A-Dimethacrylat, Ethylenglycoldiacrylat, Polyethylenglycoldiacrylat bildende Monomere, Butandioldiacrylat, Trimethylolpropantrimethacrylat, Tetraethylenglycoldiacrylat, Bisphenol-A-Diacrylat, Diethylenglycoldiacrylat, Hexandioldiacrylat, Decandioldiacrylat, Dodecandioldiacrylat, Trimethylolpropantriacrylat, deren Derivaten und Gemischen dieser Verbindungen, insbesondere BIS-GMA und TEDMA, Gemischen dieser Verbindungen oder Urethandimethacrylat und/oder Tetraethylenglycoldimethacrylat gebildet wird.

Das Gemisch kann vorteilhaft auch einen vorzugsweise nicht-kristallinen Füllstoff umfassen, der insbesondere aus Quarz, kristallfreiem hochdispersem Siliziumdioxid, Aluminiumoxid, Keramik, Glas, silanisiertem Glaspulver, Glaskeramik, Barium-Glas und Gemischen dieser Füllstoffe gewählt wird. Dabei ergibt sich aus der vorliegenden Offenbarung für den Fachmann, dass als Vergussmassen beliebige andere Vergussmassen in Betracht kommen, sofern diese die für den jeweiligen Anwendungsfall benötigten Eigenschaften, bei medizinischen Instrumenten also insbesondere Beständigkeit beim Reinigen, Desinfizieren und Sterilisieren, aufweisen. Weitere besonders geeignete Vergussmassen sind aus der WO 2007 090387 A2, aber auch aus der bereits genannten WO 2011 054355 A2 bekannt, auf die insofern als allgemeines Fachwissen verwiesen werden kann.

Nach dem Aushärten der Vergussmasse können die von dem Abschnitt 10 etwaig vorspringenden Bereiche der ausgehärteten Vergussmasse in an sich bekannter Weise so bearbeitet, z.B. beschliffen, werden, dass der Abschnitt wieder seine ursprüngliche Form annimmt, also so aussieht, als wäre keine Nut ausgebildet worden. Dazu kann die Vergussmasse farblich an den Abschnitt des zu markierenden Objektes, in dem die Nut ausgebildet wird, angepasst sein. Sie kann aber auch eine andere Farbe als der Abschnitt besitzen und/oder partiell transparent sein, so dass das Funketikett sichtbar bleibt. Dabei können bewusst bestimmte Farben eingesetzt werden, die es auch ohne Auslesen des Funketiketts 60 erlauben, das markierte Objekt, also z.B. ein medizinisches Instrument, einem bestimmten Satz von Objekten zuzuordnen und so eine Vorsortierung zu ermöglichen.

Eine Besonderheit der hinterschnittenen Nut 12 ist, dass ihr in Fig. 5 in seiner Gesamtheit mit 22 bezeichneter Boden in ihrer durch den strichpunktierten Doppelpfeil 24 in Fig. 5 angedeuteten Längsrichtung mit wenigstens zwei in der Längsrichtung gegenläufigen Gefällen ausgebildet ist. Vorteilhaft erfolgt das Einbringen der Nut 12 in das Objekt seitlich-horizontal gesehen zur Längsachse 24 wellenartig, so dass die beiden gegenläufigen Gefälle von wenigstens zwei in der Längsrichtung versetzt nebeneinander verlaufenden Wellenbahnen 26 und 28 gebildet werden. Anders ausgedrückt verläuft die Nut 12 in sich gedreht auf der einen Seite nach hinten abgesenkt (angedeutet durch die rein beispielhaft genannten +2° nach unten links in Fig. 8 - vgl. auch Fig. 6) und auf der anderen Seite noch vorne abgesenkt (angedeutet durch die rein beispielhaft genannten -2° nach oben rechts in Fig. 8 - vgl. auch Fig. 7) in verschiedenen Achsen. Vorteilhaft kann der Boden 22 angeraut sein. Insgesamt bewirkt die Ausgestaltung des Bodens, dass der nach dem Aushärten eines darin eingebrachten Gemischs erzeugte Vergussblock, der das Funketikett enthält, in allen Richtungen formschlüssig gesichert ist und bei Auftreten von Kräften in der Längsrichtung gegen die zueinander geneigten Seitenwände 18, 20 gedrückt wird, was ein Herausschieben des Vergussblocks aus der Nut zuverlässig verhindert.

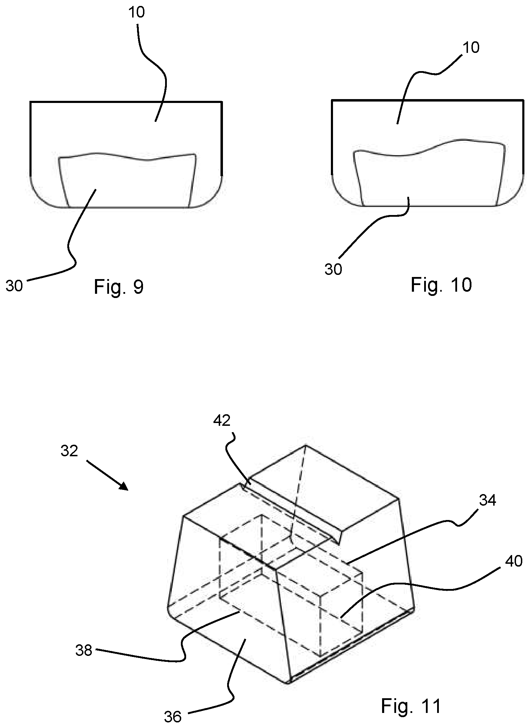

In den Figuren 9 und 10 ist ein Abschnitt 10 eines anderen zu markierenden Objektes gezeigt, einmal in einer Draufsicht ähnlich Fig. 8 auf eine erste Seite des Abschnittes 10 des Objektes, einmal in einer Draufsicht auf eine der ersten Seite gegenüberliegende Seite des Abschnittes 10 des Objektes, bei dem die Nut nach Einbringen eines Funketiketts mit Vergussmasse ausgefüllt und die Vergussmasse ausgehärtet wurde, so dass sich der das Funketikett enthaltenden Vergussblock 30 bildet. Je nachdem, wie die Vergussmasse eingebracht wird, kann ein Beschleifen des Vergussblocks 30 und ggf. des Objektes nach dem Aushärten der Vergussmasse erfolgen, um eine durchgehend bündige Oberfläche von Objekt und Vergussblock 30 zu bilden.

Fig. 11 zeigt einen in seiner Gesamtheit mit 32 bezeichneten Hüllkörper zur Aufnahme eines Funketiketts und zum Einbringen zusammen mit einem darin aufgenommen Funketikett in eine wie vorstehend beschrieben ausgebildete Nut. Bei dem Hüllkörper 32 kann es sich zum Beispiel um ein Spritzgussteil handeln.

Der Hüllkörper 32 verfügt über eine bei diesem Ausführungsbeispiel quaderförmige, durch gestrichelte Linien angedeutete Ausnehmung 34 für ein Funketikett. Die Ausnehmung 34 ist in der dargestellten Lage zum Boden 36 des Hüllkörpers 32 hin offen, wobei vorteilhaft zumindest einander gegenüberliegende Kanten 38 und 40 der Ausnehmung vorteilhaft mit Vorsprüngen versehen sein können, die ein in die Ausnehmung 34 eingebrachtes Funketikett in der Ausnehmung 34 zurückhalten, so dass es dort nicht weiter verklebt werden muss.

Bei dem gezeigten Ausführungsbeispiel ist der Hüllkörper 32 mit einer Ausrichtmarkierung 42 in Form einer Keilnut versehen, die eine Vorzugsrichtung beim Einbringen des Hüllkörpers in eine Nut in einem zu markierenden Objekt angibt. Eine solche Markierung 42 kann leicht nicht nur von einem Menschen, sondern auch von einer Maschine erkannt werden und nicht nur dazu beitragen, eine gewünschte Ausrichtung des Funketiketts in der Nut zu gewährleisten, sondern auch dazu, den Fertigungsvorgang weiter zu automatisieren. Generell hat das Verwenden eines Hüllkörpers 32 verschiedene Vorteile, insbesondere erleichtert er die Handhabung der in der Regel sehr kleinen Funketiketten und ermöglicht z.B. ein maschinelles Greifen und Einbringen eines Funketiketts, wenn dieses zuvor in einen Hüllkörper 32 eingebracht wurde. Zudem bewirkt der Hüllkörper, dass entsprechend seinem Volumen weniger Vergussmasse in die Nut zum Ausgießen selbiger eingebracht werden muss, was den Vorgang des Ausgießens erleichtert und beschleunigt.

Typischerweise besitzen Funketiketten bevorzugte Abstrahlrichtungen und sollten daher in einer definierten Weise in eine Nut in einem zu markierenden Objekt eingebracht werden. Die Ausrichtmarkierung 42 dient aber nicht nur dazu, eine solche Vorzugsrichtung anzugeben, sondern auch dazu, zu erkennen, welche Seite beim Einbringen des Hüllkörpers 32 in eine Nut in einem zu markierenden Objekt dem Boden der Nut abgewandt bzw. zugewandt sein sollte.

Bei dem dargestellten Ausführungsbeispiel würde der Hüllkörper 32 samt Funketikett so in die Nut eingebracht, dass die Ausrichtmarkierung 42 parallel zur Längsrichtung der Nut verläuft und die Seite des Hüllkörpers mit der Ausrichtmarkierung dem Boden der Nut gegenüberliegt. Bei dem gezeigten Ausführungsbeispiel wäre dann sichergestellt, dass sich das Funketikett nahe dem Boden der Nut befindet, was sich positiv auf das Sende- und Empfangsverhalten des Funketiketts auswirkt. Diese Ausgestaltung erlaubt es, bei ähnlichen zu markierenden Objekten, z.B. medizinischen Instrumenten, die Funketiketten immer in nahezu identischem Abstand zum Boden des zu markierenden Objektes anzubringen, was eine gleichbleibende zuverlässige Lesefunktionsfähigkeit ohne größere Schwankungen der Lesequalität begünstigt.

Mit Hüllkörpern 32 kann das Einbringen von Funketiketten durch ein standardisiertes Robotikverfahren automatisiert werden, so dass auch große Mengen von Objekten schnell markiert werden können. Allgemein kann, ähnlich wie vorstehend bereits erwähnt, so vorgegangen werden, dass der Hüllkörper in der Nut angeklebt wird, bevor die Nut mit Vergussmasse ausgefüllt wird.

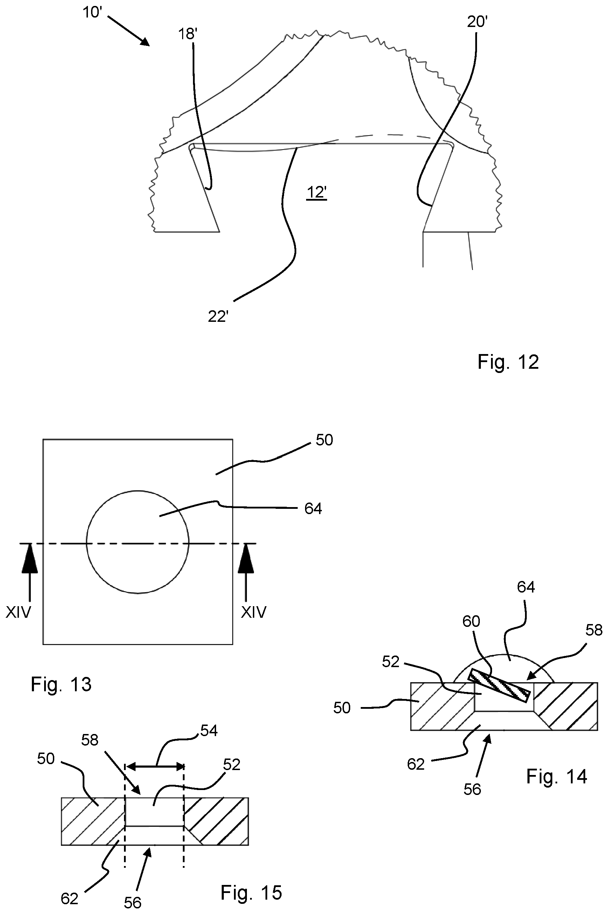

Fig. 12 zeigt ähnlich Fig. 8 eine Seitenansicht eines Abschnitt eines Objektes 10' mit einer Nut 12' gemäß einer weiteren Ausführungsform, die nicht Gegenstand dieser Anmeldung ist. Deutlich zu erkennen ist, dass der Boden 22' in sich gewellt ist. Gleichwohl weist er wenigstens zwei in der Längsrichtung gegenläufige Gefälle auf. Seitlich wird die Nut 12' wiederum von zwei zueinander geneigten Seitenwänden 18' und 20' begrenzt. Gut zu erkennen ist, dass die Übergänge von den Seitenwänden 18', 20' zum Boden 22' abgerundet sind, also keinen spitzen Winkel bilden, was vorteilhaft das vollständige Ausgießen der Nut 12' mit einer entsprechenden Vergussmasse begünstigt, die so leicht auch in die Übergangsbereiche zwischen den Seitenwänden 18', 20' und dem Boden 22' eindringen kann. Auch die in den Fig. 8 gezeigte Nut 12 ist so ausgebildet

In den Figuren 13 bis 15 ist ein Abschnitt 50 eines erfindungsgemäß markierten Objektes gezeigt, wobei die Figuren 13 und 14 den Abschnitt 50 im fertig markierten Zustand, d.h. einschließlich eines Funketiketts und ausgehärteter Vergussmasse zeigen, während Fig. 15 den Abschnitt 50 gemäß Fig. 14 ohne Funketikett und Vergussmasse zeigt. Zur Vermeidung einer Überfrachtung der Zeichnungen wurden in den Figuren nicht jeweils alle Teile mit Bezugszeichen versehen.

In den Abschnitt 50 ist eine Durchgangsbohrung 52 mit einem durch den Doppelpfeil angedeuteten Durchgangsdurchmesser 54 und zwei einander gegenüberliegenden Öffnungen 56, 58 vorgesehen ist, wobei bei diesem Ausführungsbeispiel eine erste Öffnung 56 der beiden Öffnungen durch sog. Senken (manchmal auch Ansenken genannt) kegelförmig gegenüber dem Durchgangsdurchmesser verbreitert ist.

In der in den Figuren 13 und 14 gezeigten Situation ist ein Funketikett 60 partiell so in der Durchgangsbohrung 52 im Bereich der zweiten Öffnung 58 der beiden Öffnungen angeordnet, dass in Richtung der Durchgangsbohrung 52 gesehen ein Abschnitt des Funketiketts 60 über den Durchgangsdurchmesser 54 der Durchgangsbohrung 52 hinausragt. Das Funketikett 60 und die Durchgangsbohrung 52 einschließlich ihrer Öffnungen 56, 58 und der kegelförmigen Verbreiterung 62 der ersten Öffnung 56 wurde mit einer in Fig. 14 durchsichtig und nicht schraffiert dargestellten Vergussmasse vergossen, die nach Aushärten zusammen mit dem Funketikett 60 einen äußerst stabilen Vergussblock und eine Schutzschicht in Form eines Kugelsegments 64 über dem Funketikett 60 über der zweiten Öffnung 58 bildet. Anders gesagt sind in der in den Figuren 13 und 14 gezeigten Situation das Kugelsegment 64, die Durchgangsbohrung 52, deren Öffnungen 56 und 58 einschließlich der kegelförmigen Verbreiterung 62 vollständig mit Vergussmasse ausgefüllt, die das Funketikett 60 vollständig einhüllt.

Da das Funketikett wie insbesondere in Fig. 14 gut zu erkennen über den Durchgangsdurchmesser 54 der Durchgangsbohrung 52 hinausragt, trägt es zur sicheren Halterung des sich nach Aushärten der Vergussmasse ergebenden Vergussblocks in dem zu markierenden Objekt dadurch bei, dass es den Vergussblock gegen ein Herausdrücken in Richtung der Verbreiterung 62 sichert, wozu natürlich auch das Kugelsegment 64 beiträgt, während die Verbreiterung 62, genauer gesagt die in dieser Verbreiterung befindliche ausgehärtete Vergussmasse den Vergussblock gegen ein Herausdrücken in Richtung der zweiten Öffnung 58 sichert. Je nach Ausgestaltung des Objektes und insbesondere dessen Materialstärke, kann es vorteilhaft sein, dass Funketikett 60 nicht in der zweiten Öffnung 58 zu platzieren, sondern quer über selbige zu legen. In einem solchen Fall würden dann sogar zwei Abschnitte des Funketiketts 60 über den Durchgangsdurchmesser der Durchgangsbohrung 52 ragen.

Je nachdem, wie die Vergussmasse eingebracht wird, kann ein Beschleifen des Vergussblocks und ggf. des Objektes nach dem Aushärten der Vergussmasse erfolgen, um auf der Seite der ersten Öffnung 56 eine durchgehend bündige Oberfläche von Objekt und Vergussblock zu bilden.

Im Rahmen des Erfindungsgedankens sind zahlreiche Abwandlungen und Weiterbildungen möglich, die sich z.B. auf die genaue Ausgestaltung der Form des Bodens der Nut gemäß einem Aspekt der Erfindung beziehen. Wesentlich bei diesem Aspekt ist, dass der Boden der Nut so geformt ist, dass sich nach dem Aushärten der Vergussmasse eine den Vergussblock in alle Raumrichtungen sichernde formschlüssige Verbindung ergibt. Die Nut kann vor dem Einbringen der Vergussmasse bearbeitet, insbesondere angeraut werden. Durch ein geeignetes Werkzeug kann ein solches Anrauen auch in einem Arbeitsgang mit der Einbringung der erfolgen. Ein Anrauen begünstigt die Haltbarkeit der Klebeverbindungen. Je nach Form und Größe des Funketiketts kann diesem gemäß einem anderen Aspekt der Erfindung ganz oder (wie in den Figuren als Beispiel gezeigt) teilweise in der Durchgangsbohrung angeordnet werden. Wenn ein Funketikett ganz in der Durchgangsbohrung angeordnet werden kann, können beide Öffnungen der Durchgangsbohrung gesenkt werden, wobei dann die in den jeweiligen kegelförmigen Verbreiterungen ausgehärtete Vergussmasse ein Herausdrücken des Vergusskörpers einschließlich des Funketiketts zur jeweils gegenüberliegende Seite verhindert.

### BEZUGSZEICHENLISTE

- 10, 10': Abschnitt
- 12, 12': Nut
- 14: Schenkel
- 16: Auge
- 18, 18': Seitenwand
- 20, 20': Seitenwand
- 22, 22': Boden
- 24: Längsrichtung
- 26: Wellenbahn
- 28: Wellenbahn
- 30: Vergussblock
- 32: Hüllkörper
- 34: Ausnehmung
- 36: Boden
- 38: Kante
- 40: Seite Rücksprung
- 42: Ausrichtmarkierung
- 50: Abschnitt
- 52: Durchgangsbohrung
- 54: Durchgangsdurchmesser
- 56: Öffnung
- 58: Öffnung
- 60: Funketikett
- 62: kegelförmige Verbreiterung
- 64: Kugelsegment

## Patentansprüche

1. Verfahren zum Markieren eines Objektes mit einem Funketikett, umfassend die Schritte:
- Ausbilden einer Durchgangsbohrung (52) mit einem Durchgangsdurchmesser (54) und zwei einander gegenüberliegenden Öffnungen (56, 58) in einem Abschnitt (50) des zu markierenden Objektes,
- Senken wenigstens einer ersten (56) der beiden Öffnungen (56, 58) zur kegelförmigen Verbreiterung (62) der Öffnung (56) gegenüber dem Durchgangsdurchmesser (54),
- Anordnen eines Funketiketts (60) zumindest partiell in der Durchgangsbohrung (52),
- Vergießen des Funketiketts (60) und der Durchgangsbohrung (52) einschließlich ihrer Öffnungen (56, 58) und jeder kegelförmigen Verbreiterung (62) mit einer aushärtbaren Vergussmasse unter Ausbildung einer Schutzschicht (64) über dem Funketikett (60) und
- Aushärten der Vergussmasse zur Bildung eines Vergusskörpers.

2. Verfahren nach Anspruch 1, **dadurch gekennzeichnet, dass** beide Öffnungen (56, 58) zur kegelförmigen Verbreiterung (62) gesenkt werden.

3. Verfahren nach Anspruch 1 oder 2, **dadurch gekennzeichnet, dass** das Funketikett (60) in oder über der zweiten (58) der beiden Öffnungen (56, 58) derart angeordnet wird, dass in Richtung der Durchgangsbohrung (52) gesehen wenigstens ein Abschnitt des Funketiketts (60) über den Durchgangsdurchmesser (54) hinausragt.

4. Verfahren nach einem der Ansprüche 1 bis 3, **dadurch gekennzeichnet, dass** die Schutzschicht (64) kugelsegmentartig über dem Funketikett (60) ausgebildet wird.

5. Mit einem Funketikett (60) markiertes Objekt, das einen Abschnitt (50) aufweist, in dem eine Durchgangsbohrung (52) mit einem Durchgangsdurchmesser (54) und zwei einander gegenüberliegenden Öffnungen (56, 58) vorgesehen ist, **dadurch gekennzeichnet, dass**
- wenigstens eine erste (56) der beiden Öffnungen (56, 58) kegelförmig gegenüber dem Durchgangsdurchmesser (54) verbreitert ist,
- ein Funketikett (60) zumindest partiell in der Durchgangsbohrung (52) angeordnet ist, und
- das Funketikett (60) und die Durchgangsbohrung (52) einschließlich ihrer Öffnungen (56, 58) und jeder kegelförmigen Verbreiterung (62) mit einer ausgehärteten Vergussmasse vergossen ist, die eine Schutzschicht (64) über dem Funketikett (60) bildet.

6. Objekt nach Anspruch 5, wobei beide Öffnungen (56, 58) gegenüber dem Durchgangsdurchmesser (54) kegelförmig verbreitert sind.

7. Objekt nach Anspruch 5 oder 6, **dadurch gekennzeichnet, dass** das Funketikett (60) in oder über der zweiten (58) der beiden Öffnungen (56, 58) derart angeordnet ist, dass in Richtung der Durchgangsbohrung (52) gesehen wenigstens ein Abschnitt des Funketiketts (60) über den Durchgangsdurchmesser (54) hinausragt.

8. Objekt nach einem der Ansprüche 5 bis 7, **dadurch gekennzeichnet, dass** die Schutzschicht (64) kugelsegmentartig über dem Funketikett (60) ausgebildet ist.
